(19) Europäisches Patentamt
European Patent Office
Office européen des brevets

(11) EP 1 816 115 A2

(12) **EUROPEAN PATENT APPLICATION**

(43) Date of publication:
**08.08.2007 Bulletin 2007/32**

(21) Application number: **06026997.4**

(22) Date of filing: **28.12.2006**

(51) Int Cl.:
***C07C 41/26*** *(2006.01)* ***C07C 43/13*** *(2006.01)*
***C08G 65/00*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **28.12.2005 JP 2005377418**

(71) Applicant: **Kao Corporation**
**Chuo-ku**
**Tokyo 103-8210 (JP)**

(72) Inventors:
- **Shirasawa, Takeshi**
  **Wakayama-shi**
  **Wakayama 640-8580 (JP)**
- **Kono, Yuichi**
  **Wakayama-shi**
  **Wakayama 640-8580 (JP)**

(74) Representative: **HOFFMANN EITLE**
**Patent- und Rechtsanwälte**
**Arabellastrasse 4**
**81925 München (DE)**

(54) **Method for producing glycidiyl ether**

(57) A method for producing a reaction product using a reaction system in which an organic compound and water react with each other to generate the reaction product and furthermore part of the reaction product consecutively reacts. A filler for regulating the consecutive reaction of the reaction product is provided in a reaction field of a fluid mixture of the organic compound and the subcritical water.

FIG. 1A

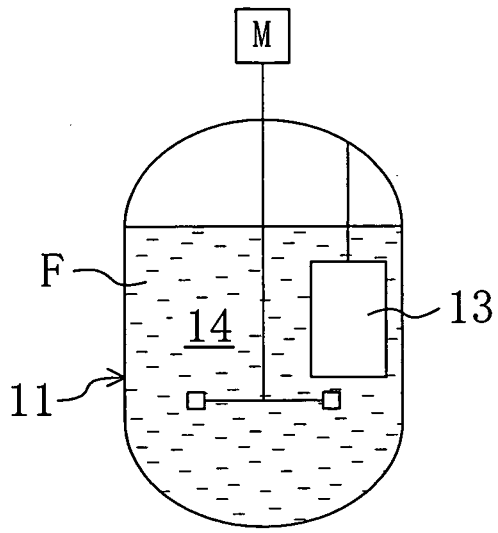


EP 1 816 115 A2



(Cont. next page)

FIG. 1B

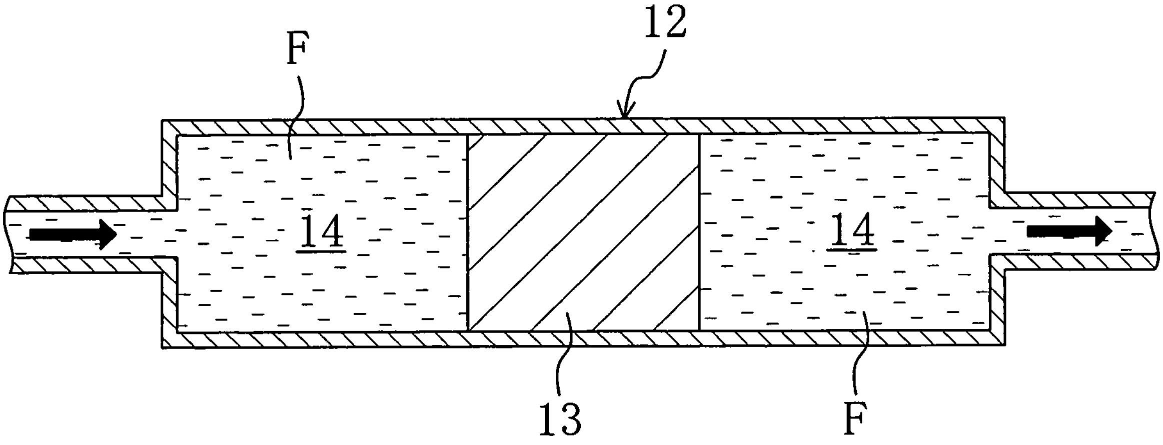

## Description

## FIELD OF THE INVENTION

The present invention relates to a method for producing a reaction product using a reaction system in which an organic compound and water react with each other to generate the reaction product and furthermore part of the reaction product consecutively reacts.

## BACKGROUND OF THE INVENTION

As methods for producing glyceryl ether, a method in which a three-membered ring of glycidyl ether is opened with the existence of a base catalyst or an acid catalyst, a method in which a three-membered ring of glycidyl ether is opened using carboxylic acid and a base as catalysts, a method in which glycidyl ether is changed to oxolane and then oxolane is hydrolyzed and like methods have been known.
Japanese Patent Publication No. 2002-88000 discloses a method for producing a polyhydroxy compound in which epoxide is hydrolyzed with a subcritical water. According to this method, without needing a catalyst and a solvent, a polyhydroxy compound can be efficiently and selectively obtained by the hydrolysis of epoxide.
Japanese Patent Publication No. 2003-267901 discloses a method for producing glyceryl ether in which a hydrolysis reaction is performed while glycidyl ether having a specific structure and water are continuously supplied to a reactor and, furthermore, water is separately recovered from a reaction mixture exhausted from the reactor and circulated in the reactor. According to this method, operation and reaction control can be performed in a simple manner. Therefore, there is only a small amount of loss of water used during the reaction and glyceryl ether can be produced with a high yield.
Japanese Patent Publication No. 2003-267902 discloses a method for producing glyceryl ether in which glycidyl ether having a specific structure is hydrolyzed within a temperature range of 0 °C or more and less than 250 °C without using any catalyst. According to this method, a product of the reaction does not contain unnecessary concomitants such as ions, and a reaction product can be purified in a simple manner and glyceryl ether can be produced with a high yield.

## SUMMARY OF THE INVENTION

The present invention provides a method for producing a reaction product using a reaction system in which an organic compound and water react with each other and furthermore part of the reaction product consecutively reacts. In the method, a filler is provided in a reaction field of a fluid mixture of the organic compound and the subcritical water.

## BRIEF DESCRIPTION OF THE DRAWINGS

FIG. **1A** is a view of a vessel type reactor and FIG. **1B** is a view of a continuous reactor.

## DETAILED DESCRIPTION OF THE INVENTION

The present invention may make it possible to obtain a reaction product with a high yield. According to the present invention, the filler is provided in the reaction field. Thus, the consecutive reaction of the reaction product is regulated, so that loss caused by the consecutive reaction is suppressed. As a result, the reaction product can be obtained with a high yield.
Other features and advantages of the present invention will be apparent from the following description with reference to the accompanying drawings.
Hereafter, embodiments of the present invention will be described in detail.
A method for producing a reaction product according to an embodiment uses a reaction system in which an organic compound and water react with each other to generate the reaction product and part of the reaction product consecutively reacts. In the method, a filler for regulating the consecutive reaction of the reaction product is provided in a reaction field of a fluid mixture of the organic compound and the subcritical water.
According to this method, a filler for regulating the consecutive reaction of a reaction product is provided in a reaction field. Thus, loss caused by the consecutive reaction of a reaction product can be suppressed, so that the reaction product can be obtained with a high yield.
The method for producing a reaction product according to this embodiment uses a reaction system in which an organic compound and water react with each other to generate the reaction product and furthermore part of the

reaction product consecutively reacts.

As the reaction system of this embodiment, i.e., a reaction system in which an organic compound and water react with each other to generate a reaction product and furthermore part of the reaction product consecutively reacts, for example, there are ring-opening reactions of a three-membered ring of epoxide (i.e., an organic compound including cyclic ether with a three-membered ring containing oxygen as one of its atoms), hydration reactions of an organic compound including a carbon double bond within a molecule or like method is used. In a reaction system for the ring-opening reaction of a three-membered ring of epoxide, diol is obtained as a reaction product and part of the diol as a reaction product consecutively reacts with epoxide to generate ether. Moreover, in a reaction system for the hydration reaction of an organic compound including a carbon double bond within a molecule, alcohol is obtained as a reaction product and part of the alcohol as a reaction product consecutively reacts, thus generating dimer ether.

As an organic compound used as a material, for example, an organic compound including an ether bond such as epoxide, a carbon double bond, an ester bond, an amide bond or the like within a molecule is used.

Specifically, this method is preferable to the case where glycidyl ether expressed by the following general formula (Formula 1) is used as an organic compound.

[Formula 1]

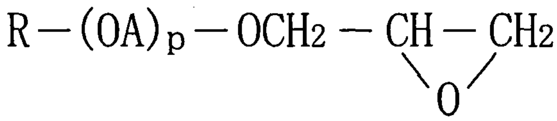

(I)

As a hydrocarbon group represented by **R,** for example, an alkyl group including a normal chain or a branched chain containing 1 to 20 carbon atoms, an alkenyl group including a normal chain or a branched chain containing 2 to 20 carbon atoms, an aryl group containing 6 to 14 carbon atoms or the like is used. Note that in a hydrocarbon group represented by **R,** part or all of the hydrogen atoms may be substituted by fluorine atoms. The hydrocarbon group represented by **R** preferably contains 1 to 12 carbon atoms, whereby excellent reactivity and reaction selectivity can be obtained.

**OA** stands for an oxyalkylene group. In this case, a plurality of the same or different oxyalkylene groups may be contained. As an alkylene group represented by A, which contains 2 to 4 carbon atoms, for example, an ethylene group, a trimethylene group, a propylene group, a butylene group or the like is used. **p** is 0 to 20 and is preferably 0.

Specifically, as R-(OA)p-, for example, a methyl group, an ethyl group, a n-propyl group, a n-butyl group, a n-pentyl group, a n-hexyl group, a n-heptyl group, a n-octyl group, a n-nonyl group, a n-decyl group, a n-dodecyl group, a tetradecyl group, a hexadecyl group, an octadecyl group, an eicocyl group, a 2-propyl group, a 2-butyl group, a 2-methyl-2-propyl group, a 2-pentyl group, a 3-pentyl group, a 2-hexyl group, a 3-hexyl group, a 2-octyl group, a 2-ethylhexyl group, a phenyl group, a benzyl group, an alkylene oxide adduct (such as ethylene oxide and propylene oxide) of any one of the above-described groups or the like is used.

Moreover, as examples of $R\text{-}(OA)_p\text{-}$ in which hydrogen atoms of the hydrocarbon group represented by **R** are substituted by fluorine atoms, for example, a perfluoroalkyl group such as a nanofluorihexyl group, a hexafluorohexyl group, a tridecafluorooctyl group, a heptafluorooctyl group, a heptadecafluorodecyl group and the like, an alkylene oxide adduct of any one of the above-described groups, or the like is used. The degree and site of substitution of hydrogen atoms by fluorine atoms are not particularly limited.

As water used as a material, for example, ion-exchanged water, distilled water, reverse osmosis treated water or the like is used. Water may contain salts such as an acid and a base in accordance with the intended use.

As water used as a material, for example, ion-exchanged water, distilled water, reverse osmosis treated water or the like is used. Water may contain salts such as an acid and a base in accordance with an intended use.

Accordingly, as a reaction product, for example, diol obtained from an organic compound including an ether band such as epoxide within a molecule, alcohol obtained from an organic compound including a carbon double bond within a molecule, a hydrolysate obtained from an organic compound including an ester bond, an amide bond within a molecule or the like is used.

When glycidyl ether is used as an organic compound, glyceryl ether is obtained as a reaction product. As glyceryl ether, for example, n-butyl glyceryl ether, 2-methylpropyl glyceryl ether, n-pentyl glyceryl ether, 2-methylbutyl glyceryl ether, n-hexyl glyceryl ether, 2-methylpentyl glyceryl ether, phenyl glyceryl ether, n-octyl glycidyl ether, 2-ethylhexyl glyceryl ether, n-stearyl glyceryl ether or the like is used.

The method for producing a reaction product according to this embodiment, a filler for regulating the consecutive reaction of the reaction product is provided in a reaction field of a fluid mixture of an organic compound and a subcritical water.

Herein, "subcritical water" means water in a liquid state at a temperature of 100 °C (boiling point) or more and less than 374 °C (critical point temperature).

A temperature of the reaction field is preferably 150 °C or more and less than 374 °C and, in view of smoothing the generation of a reaction product and reducing a load on facility and equipment, is more preferably 200 to 300 °C.

In the reaction field, a higher pressure than a saturated vapor pressure is normally loaded. However, a pressure loaded in the reaction filed is preferably from about 0.1 to 30 MPa.

In the fluid mixture of an organic compound and a subcritical water, a stoichiometry amount of subcritical water with respect to an organic compound differs depending on the type of the organic compound. For example, assume that the organic compound is glycidyl ether. In view of suppressing the progress of consecutive reaction of a reaction product and achieving high yield production of the reaction product, the stoichiometry amount of the subcritical water with respect to the organic compound is preferably 2 to 500 and more preferably 10 to 100.

The residence time, i.e., the reaction time in the reaction field varies depending on the type of the organic compound and the temperature of the reaction field. In general, the residence time ranges from 3 minutes to 10 hours. For example, when glycidyl ether as the organic compound is brought into reaction, the reaction time is about 10 minutes.

The filler provided in the reaction field may be in a bulk form, a wire form, a flake form, a plane form or the like. More specifically, as the filler, for example, round beads used in a beads mill; a random packing such as McMahon packing and Dickson packing used in fractionating; a structured packing such as Sulzer packing (available from Sulzer Chemtech, Ltd.), Montz pak (available from Montz), Mellapak (available from Sulzer Chemtech, Ltd.), Technopak (available from Mitsui & Co., Ltd.), ROMBOPAK (available from Tsukishima Kikai Co., Ltd.), NK Pack (available from Japan Chemical Engineering & Machinery Co., Ltd.), and Goodroll packing (available from Tokyo Special Wire Netting Co, Ltd.) used in fractionating; a porous material such as a honeycomb material; a fiber assembly such as a nonwoven fabric, cotton and the like; or the like is used.

A material for the filler, for example, metal materials including steel, Fe-Cr-Ni alloy such as stainless, carpenter 20 and the like, copper alloy, aluminum alloy, Ni-Cr-Fe alloy, Ni-Cu alloy, Ni-Mo-Fe-Cr alloy, cobalt alloy, titanium alloy, zirconium alloy, molybdenum, chromium and the like; hard glass; quartz glass; porcelain; glass lining; plastic; ceramics; and the like can be used. Among those materials, ones which have hydrophobic properties and of which an average contact angle with water is 30 ° or more are preferable and, furthermore, ones which have hydrophobic properties and of which an average contact angle with water is 40 ° or more are more preferable. Considering resistance in the reaction field, a metal material such as austenite stainless, Ni-Cr-Fe alloy, Ni-Mo-Fe-Cr alloy and the like are more preferable. Among them, Ni-Cr-Fe alloy and Ni-Mo-Fe-Cr alloy are particularly preferable. Moreover, the filler may be formed of a material which does not have the catalyst function in the reaction system.

Taking it into account that a larger contact area of the fluid mixture with the filler is more preferable, a surface area of the filler with respect to a volume of the reaction field is preferably 500 $m^2/m^3$ or more and is more preferably 1000 $m^2/m^3$.

The method for producing a reaction product according to this embodiment can be implemented in a batch operation in which a necessary amount of each of the materials for one batch is supplied to a reactor and an operation is performed each time, a continuous operation in which each of the materials is continuously supplied to the reactor and an operation is continuously performed, and a semi-batch operation in which one of the materials is loaded in the reactor in advance and the other material is continuously supplied to the reactor. Note that considering the easiness of controlling reaction conditions and efficiency of the reaction progress, a continuous operation is more preferable.

FIG. **1A** is a view of a batch reactor **11** and FIG. **1B** is a view of a continuous reactor **12.**

In the batch reactor **11,** as shown in FIG. **1A,** the filler **13** is provided in the reaction field **14** so as to be in contact with a fluid mixture **F.** In the continuous reactor **12,** as shown in FIG. **1B**, the filler **13** is provided in the reaction field **14** so as to break a flow path in the reactor **12** through which the fluid mixture **F** flows.

When a batch operation is performed, in the batch reactor **11,** an organic compound and water are supplied at a predetermined mixture ratio. When a continuous operation is performed, in the continuous reactor **12,** the amount of each of the organic compound and water is adjusted so that the ratio between a supply amount of the organic compound per unit time and a supply amount of water per unit time is a predetermined ratio in a steady state of reaction (i.e., a state where components relating to the reaction are constant).

In the case of a batch operation, the operating time is considered to start at a time when loading materials is completed. In the case of a continuous operation, the operating time is considered to start at a time when reaction reaches a steady state.

As a reactor used in the method for producing a reaction product according to this embodiment, for example, a tank reactor such as an autoclave can be used in the case of a batch operation. In the case of a continuous operation, for example, a tube reactor, a tower reactor, a static mixer reactor, a semi-batch reactor or the like is used. Although a reactor with or without stirring means can be used, in view of achieving the uniform progress of a reaction, a reactor with stirring means is preferably used so as to perform the reaction while stirring. Note that each of the above-described reactors is commercially available.

In a method for producing a reaction product according to this embodiment, an organic compound and water may be mixed before being supplied to a reactor or may be separately supplied to the reactor and mixed in the reactor. In the case of a batch operation, for example, using a homomixer, a high-shear disk turbine stirring blade, a puddle tilted stirring blade or the like, an organic compound and water are mixed before or after supply of the organic compound and the water to the reactor. In the case of a continuous operation, for example, a line homomixier, a static mixer, a disper or the like can be provided.

After completion of the reaction, a mixture containing an obtained reaction product is recovered and cooled down. Then, according to a known method, the mixture is purified by vaporization or distillation, spontaneous or centrifugal separation or the like to separate the reaction product from unreacted water. Thus, the reaction product can be obtained.

The following examples further describe and demonstrate embodiments of the present invention. The examples are given solely for the purpose of illustration and are not to be construed as limitations of the present invention.

[Implement example]

(Examination contents)

Examinations were performed in the manner described in implementation examples and comparison examples. Details of the examinations will be described in Table 1.

[Table 1]

| | | Implementation example 1 | Comparison example 1 | Implementation example 2 | Comparison example 2 |
|---|---|---|---|---|---|
| Reaction temperature (°C) | | 250 | 250 | 270 | 270 |
| Flow rate (mL/min) | Glycidyl ether | 0.70 | 0.70 | 0.70 | 0.70 |
| | Water | 2.40 | 2.40 | 0.61 | 0.61 |
| Tube-type reactor | Volume (mL) | 14.7 | 14.7 | 14.7 | 14.7 |
| Filler | With or without | With | Without | With | Without |
| | Area ($m^2$) | 0.0154 | - | 0.0154 | - |
| Surface area of filler / reaction field (reaction tube) volume ($m^2/m^3$) | | 1048 | - | 1048 | - |

<Implementation example 1>

A tube-type reactor formed of an SUS316 tube was prepared. In the tube-type reactor, a filler formed of a SUS mesh (of which an average contact angle with water was 60 degrees), i.e., McMahon packing, having a line diameter of 0.1 mm and a gravity of 7.75 g/mL was provided so as to break the flow path of a fluid. The tube-type reactor had an outer diameter of 19.1 mm, an inner diameter of 14.8 mm, a length of 85 mm and an inner volume of 14.7 mL. The filler had a surface area of 0.0154 $mm^2$. Accordingly, the surface area of the filler to the volume of a reaction filed was 1048 $m^2/m^3$.

The tube-type reactor was immersed in an oil bath at a temperature of 250 °C and 2-ethylhexyl glycidyl ether (special grade available from Tokyo Chemical Industry Co., LTD.) and ion-exchanged water were continuously supplied. A flow rate of 2-ethylhexyl glycidyl ether was 0.70 ml/ min and a flow rate of the ion-exchanged water was 2.40 mL/min. Then, a reactant obtained in the tube-type reactor was cooled down and recovered via a back pressure regulating valve.

After a lapse of 4 hours from the start of reaction, a composition of a recovered material became constant and was in a steady state. The material recovered at this time was obtained as a sample.

<Comparison example 1>

Except for use of a tube-type reactor in which a filler was not provided, a sample of the recovered material was obtained in the same manner as in Implementation example 1.

<Implementation example 2>

A tube-type reactor having the same configuration as that of Implementation example 1 was immersed in an oil bath at a temperature of 270 °C and 2-ethylhexyl glycidyl ether (special grade available from Tokyo Chemical Industry Co., LTD.) and ion-exchanged water were continuously supplied. A flow rate of 2-ethylhexyl glycidyl ether was 0.70 ml/min and a flow rate of the ion-exchanged water was 0.61 mL/min. Then, a reactant obtained in the tube-type reactor was cooled down and recovered via a back pressure regulating valve.

After a lapse of 4 hours from the start of reaction, a composition of the recovered material became constant and was in a steady state. A material recovered at this time was obtained as a sample.

<Comparison example 2>

Except for use of a tube-type reactor in which a filler was not provided, a sample of the recovered material was obtained in the same manner as in Implementation example 2.

(Examination evaluation method)

The recovered materials of Implementation examples 1 and 2 and Comparison examples 1 and 2 were analyzed and evaluated by internal standard methods using gas chromatography analysis.

Using a mole fraction of the organic compound (2-ethylhexyl glycidyl ether) which disappeared through the reaction as a conversion rate, selectivity was calculated based on the following formula. A product obtained from the calculation was assumed to yield:

$$(\text{Selectivity} \ [\%]) = (\text{single adduct} \ [\text{mol}]) / (\text{single adduct} \ [\text{mol}] + \text{consecutive adduct} \ [\text{mol}] \times 2)$$

where a single adduct is glycidyl ether which is a reaction product and a consecutive adduct is ether of dimer glycidyl ether.

(Examination evaluation results)

Table 2 shows the examination evaluation results.

[Table 2]

| | Implementation example 1 | Comparison example 1 | Implementation example 2 | Comparison example 2 |
|---|---|---|---|---|
| Conversion rate (mol%) | 68.7 | 68.0 | 90.5 | 74.4 |
| Selectivity (mol%) | 97.4 | 90.2 | 95.4 | 84.9 |
| Yield | 66.9 | 61.3 | 86.3 | 63.2 |

According to the results of Table 2, from the comparison between Implementation example 1 using a tube-type reactor with a filler and Comparison example 1 using a tube-type reactor without a filler, it is understood that there is not a large difference in conversion rate but selectivity is much larger in Implementation example 1 than in Comparison example 1. Accordingly, the yield is higher in Implementation example 1 than in Comparison example 1.

In the comparison between Implementation example 2 and Comparison example 2, conversion rate and selectivity are markedly larger in Implementation example 2 than in Comparison example 2. Accordingly, yield is significantly larger in Implementation example 2 than in Comparison example 2.

The present invention is not limited to the above-described embodiments but can be implemented in various forms without departing from its spirit and essential characteristics. The above-described embodiments should be therefore be considered in all respects as illustrative only but not restrictive. The scope of the invention is indicated by the claims but not at all restricted to the description. Further, all modifications and changes which come within the range of equivalents of the claims are intended to be embraced within the scope of the invention.

## Claims

1. A method for producing a reaction product using a reaction system in which an organic compound and water react with each other to generate the reaction product and furthermore part of the reaction product consecutively reacts, wherein a filler is provided in a reaction field of a fluid mixture of the organic compound and the subcritical water.

2. The method of claim 1, wherein the fluid mixture of the organic compound and the subcritical water is continuously supplied to a fluid flow path in which the filler is provided.

3. The method of claim 1, wherein the surface area of the filler with respect to the volume of the reaction field is 500 $m^2/m^3$ or more.

4. The method of claim 1, wherein the temperature of the reaction field is 150 °C or more and less than 374 °C.

5. The method of claim 1, wherein the organic compound is glycidyl ether, expressed by the general formula of

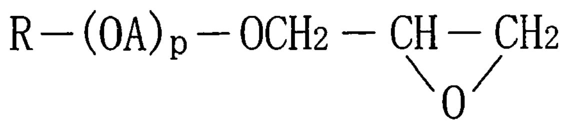

(I)

where R is a saturated or unsaturated hydrocarbon group in which part or all of the hydrogen atoms may be substituted by fluorine atoms and which has a carbon number of 1 to 20, OA is an oxyalkylene group having a carbon number of 2 to 4 and p is a number of from 0 to 20.

6. The method of claim 1, wherein the filler is formed of a material which does not have a function of serving as a catalyst in the reaction system.

7. The method of claim 1, wherein the filler is formed of metal.

FIG. 1A

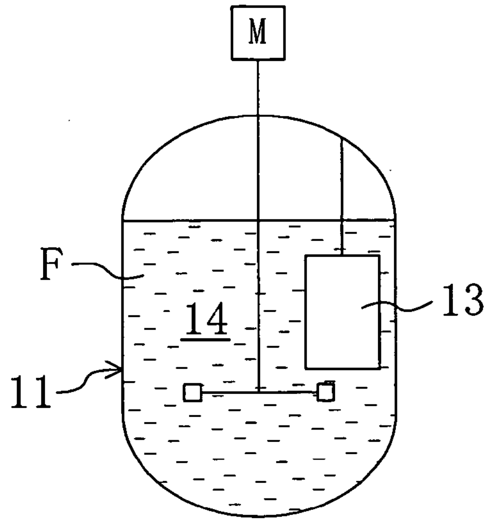


FIG. 1B

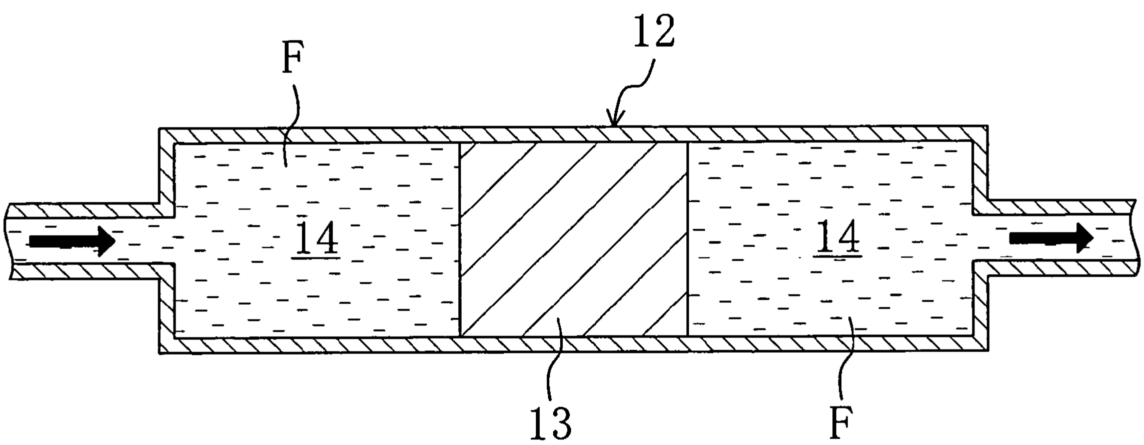

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002088000 A
- JP 2003267901 A
- JP 2003267902 A